# EUROPEAN PATENT APPLICATION

(11) **EP 3 988 100 A1**
(43) Date of publication of application: **27.04.2022**
(21) Application number: 20826924.1
(22) Date of filing: 22.06.2020
(51) Int. Cl.: A61K 31/52, C07H 19/167, A61P 9/08, A61P 9/10, A61P 37/00

(54) **ADENOSINE COMPOUND, PHARMACEUTICALLY ACCEPTABLE SALT OR STEREOISOMERIDE THEREOF, AND USE**

(30) Priority: 21.06.2019 CN 201910542120
(71) Applicant: Academy Of Military Medical Sciences, Haidian District Beijing 100850 (CN)
(72) Inventor: ZHONG, Wu, Beijing 100850 (CN); ZHANG, Min, Beijing 100850 (CN); ZHOU, Xinbo, Beijing 100850 (CN); FAN, Shiyong, Beijing 100850 (CN); LI, Song, Beijing 100850 (CN)
(74) Representative: Böhm, Brigitte
(86) International application number: PCT/CN2020/097392
(87) International publication number: WO 2020/253866

(57) **Abstract**

The present invention relates to a compound represented by Formula I, its pharmaceutically acceptable salt, its stereoisomer, its pharmaceutically acceptable hydrate or solvate, or its pharmaceutically acceptable ester, wherein R is selected from heteroaryl, substituted heteroaryl, aryl, substituted aryl, cycloalkyl, substituted cycloalkyl, alkyl, and substituted alkyl, wherein the substituted heteroaryl, the substituted aryl, the substituted cycloalkyl and the substituted alkyl are each independently substituted with one or more hydroxyalkylene groups. The compound or its pharmaceutically acceptable salt, its stereoisomer, its pharmaceutically acceptable hydrate or solvate, its pharmaceutically acceptable ester of the present invention has agonistic activity on A_{2A} adenosine receptor and can improve the permeability of blood-brain barrier to promote the delivery of drug across blood-brain barrier, and can also prevent or treat a disease associated with A_{2A} adenosine receptor agonistic activity.

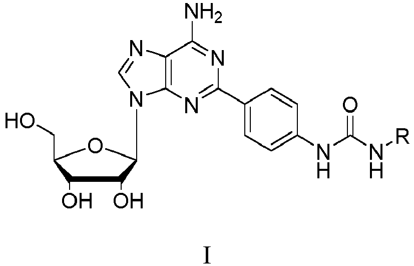

## Description

### Technical Field

The present invention belongs to the technical field of medicine, and specifically relates to an adenosine compound, its pharmaceutically acceptable salt or its stereoisomer, as well as a medical use thereof.

### Background Art

One of the main reasons for the failure of the development and research of drugs for treating central nervous system diseases is the obstruction of the blood-brain barrier (BBB). The blood-brain barrier prevents drugs from being delivered to the central nervous system and being accumulated in the brain to reach an effective dose, which affects the corresponding therapeutic effects. Therefore, one of the difficulties in the research of targeted central drugs is how to overcome the blood-brain barrier.

Finding a way for disrupting the blood-brain barrier has become a hot spot in the study of intracerebral drug delivery. In the past few decades, drug delivery across the blood-brain barrier has always been a challenging study, and researchers have made considerable efforts to this. However, a series of studies have revealed that the delivery of drug and contrast agent across the blood-brain barrier is very difficult. With the rapid increase in the demand for effective therapeutic drugs for the treatment of central nervous system diseases (e.g., brain tumors, strokes, trauma, and neurodegenerative diseases, etc.) and neurotoxic agent poisonings, it is even more important to develop drugs that can penetrate the blood-brain barrier. Therefore, it is necessary to develop an efficient blood-brain barrier disruption (BBBD) strategy, which has minimal nerve damage, can deliver larger molecular weight drugs, and has better pharmacokinetic characteristics.

Among the reasons for the obstruction of the blood-brain barrier, tight junctions (TJs) seal the endothelial cells, resulting in a low permeability of blood molecules through the BBB; on the other hand, compared with peripheral vascular endothelial cells, there are few transport pathways between cerebral vascular endothelial cells, but the expression level of active efflux transporters, such as P glycoprotein (P-gp) in brain capillary endothelial cells (BCECs), is very high. Considering the key role of TJs in restricting the entry of molecules into the brain (HUBER J D et al., Trends Neurosci, 2001, 24(12): 719-25), reversibly changing the tightness of TJs may be a feasible way to increase the permeability of BBB. Temporary opening of TJs is a feasible way to deliver drugs in the brain, because this method has high passing efficiency and less molecular weight restriction for therapeutic drugs. Bynoe et al. recently demonstrated that the specific agonizing of A_{2A} adenosine receptor (A_{2A}AR) on mouse BCECs can promote brain drug absorption (CARMAN A J et al., J Neurosci, 2011, 31(37): 13272-80). Further studies have shown that the agonizing of A_{2A} adenosine receptor can up-regulate BBB permeability and temporarily increase the intercellular space of brain capillary endothelial cells. Studies have shown that the A_{2A}AR signaling pathway modulates cytoskeletal components by regulating intracellular actin, which leads to cell morphology contraction, destruction of TJs integrity, and increased barrier permeability (SOHAIL MA et al., Hepatology, 2009, 49(1): 185-94). These studies have greatly expanded the potential application fields and development space of A_{2A}AR agonists. The development of high-efficiency A_{2A}AR agonists is of great significance to the study of the blood-brain barrier disruption strategies.

A_{2A}AR is widely distributed in the human body, and A_{2A}AR agonists are recommended for the treatment of various pathological diseases. For example, because adenosine mediates A_{2A}AR to produce potential immunosuppressive and hypotensive effects, one of the potential therapeutic effects of A_{2A}AR agonists is to exert anti-inflammatory and immunosuppressive effects by regulating the activity of neutrophils, macrophages and T lymphocytes (DE LERA RUIZ M, etc., J Med Chem, 2014, 57(9): 3623-50; VARANI K, etc., FASEB J, 2010, 24(4): 1192-204). From the perspective of cell signal pathways, the A_{2A} agonizing reduces NF-kB pathway, reduces inflammatory cytokines such as tumor necrosis factor α (TNF-α), interleukin-1β (IL-1β), IL-8, IL-6, and inhibits the release of matrix metalloproteinase-1 (MMP-1) and MMP-3 (HASKO G, etc., Nat Rev Drug Discov, 2008, 7(9): 759-70). Therefore, selective A_{2A}AR agonists have been developed to treat related diseases, such as allergic rhinitis, asthma, and chronic obstructive pulmonary disease. However, the use of A_{2A}AR agonists in anti-inflammatory drugs is limited. On the one hand, it is because while the agonizing of A_{2A}AR brings about anti-inflammatory effects, it has a strong hypotensive effect on the heart and blood vessels. Another reason is that A_{2A}AR agonists are powerful vasodilators and can be used as diagnostic reagents for cardiac pharmacological stress tests (patent: CN200580033215.2). Although A_{2A}AR agonists as powerful vasodilators can produce systemic side effects, it is reported that low doses of A_{2A}AR agonists have no significant cardiovascular side effects. In addition, further potential therapeutic applications of A_{2A}AR agonists are the treatment of psychosis and Huntington's disease (AKKARI R et al., Curr Top Med Chem, 2006, 6(13): 1375-99; BOSCH MP et al., J Med Chem, 2004, 47(16): 4041-53). Studies have shown that A_{2A}AR agonists have neuroprotective effects on neurodegenerative disease models by reducing excitatory neurotransmitter release, apoptosis and inflammation (MULLER CE, etc., Biochim Biophys Acta, 2011, 1808(5): 1290-308 ; RIVERA-OLIVER M, etc., Life Sci, 2014, 101(1-2): 1-9).

Although A_{2A}AR agonists have more and more pharmacological effects, only one A_{2A}AR agonist, regadenoson (an adenosine analog), is approved as a coronary vasodilator in the United States. Regadenoson is a selective A_{2A} adenosine receptor agonist jointly developed by CV Pharmaceutical company and Astellas company. The product has been marketed in the United States and Europe and is mainly used as a coronary vasodilator for myocardial perfusion imaging. Therefore, there is still a need in this art to study A_{2A} receptor agonists that have novel structures, are effective, and have one or more physiological and/or physicochemical advantages.

### Contents of the present invention

The present invention provides an adenosine compound, its pharmaceutically acceptable salt, its stereoisomer, its pharmaceutically acceptable hydrate or solvate, or its pharmaceutically acceptable ester, which has an agonistic activity effect on A_{2A} adenosine receptor, can improve the permeability of blood-brain barrier, thereby promoting the delivery of drugs across the blood-brain barrier, and the above-mentioned compound, its pharmaceutically acceptable salt, its stereoisomer, its pharmaceutically acceptable hydrate or solvate, or its pharmaceutically acceptable ester can also be used for the prevention or treatment of a disease related to A_{2A} adenosine receptor agonistic activity. On this basis, the present invention provides a medical use of the adenosine compound, its pharmaceutically acceptable salt, its stereoisomer, its pharmaceutically acceptable hydrate or solvate, or its pharmaceutically acceptable ester.

A first aspect of the present invention relates to a compound represented by Formula I, its pharmaceutically acceptable salt, its stereoisomer, its pharmaceutically acceptable hydrate or solvate, or its pharmaceutically acceptable ester, 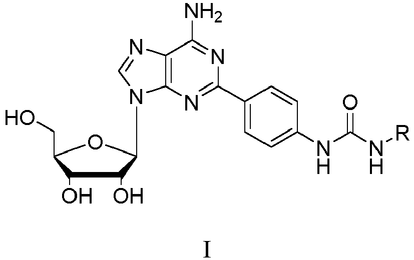 wherein, R is selected from heteroaryl, substituted heteroaryl, aryl, substituted aryl, cycloalkyl, substituted cycloalkyl, alkyl, and substituted alkyl, wherein the substituted heteroaryl, the substituted aryl, the substituted cycloalkyl and the substituted alkyl are each independently substituted with one or more (e.g., 2, 3, 4, 5) hydroxyalkylene groups.

In some embodiments of the first aspect of the present invention, R is selected from 5-to 8-membered (e.g., 6-membered, 7-membered) heteroaryl, substituted 5- to 8-membered (e.g., 6-membered, 7-membered) heteroaryl, 5- to 8-membered (e.g., 6-membered, 7-membered) aryl, substituted 5- to 8-membered (e.g., 6-membered, 7-membered) aryl, 3-to 8-membered (e.g., 4-membered, 5-membered, 6-membered, 7-membered) cycloalkyl, substituted 3- to 8-membered (e.g., 4-membered, 5-membered, 6-membered, 7-membered) cycloalkyl, C₁₋₁₀ alkyl and substituted C₁₋₁₀ alkyl, wherein the substituted 5- to 8-membered (e.g., 6-membered, 7-membered) heteroaryl, the substituted 5- to 8-membered (e.g., 6-membered, 7-membered) aryl, the substituted 3- to 8-membered (e.g., 4-membered, 5-membered, 6-membered, 7-membered) cycloalkyl and the substituted C₁₋₁₀ alkyl are each independently substituted with one or more (e.g., 2, 3, 4, 5) hydroxyl-Ci-io alkylene groups.

In some embodiments of the first aspect of the present invention, R is selected from the group consisting of heteroaryl, substituted heteroaryl, substituted aryl, and cycloalkyl, wherein the substituted heteroaryl and the substituted aryl are each independently substituted with one or more (e.g., 2, 3, 4, 5) hydroxyalkylene groups.

In some embodiments of the first aspect of the present invention, R is selected from 5-to 8-membered heteroaryl, substituted 5- to 8-membered heteroaryl, substituted 5- to 8-membered aryl and 3- to 8-membered cycloalkyl, wherein the substituted 5- to 8-membered heteroaryl and the substituted 5- to 8-membered aryl are each independently substituted with one or more (e.g., 2, 3, 4, 5) hydroxyl-Ci-io alkylene groups.

In some embodiments of the first aspect of the present invention, R is selected from the group consisting of pyrrolyl, substituted pyrrolyl, imidazolyl, substituted imidazolyl, thiazolyl, substituted thiazolyl, furyl, substituted furyl, pyridyl, substituted pyridyl, substituted phenyl and 3- to 6-membered (e.g., 4-membered, 5-membered) cycloalkyl, wherein the substituted pyrrolyl, the substituted imidazolyl, the substituted thiazolyl, the substituted furyl, the substituted pyridyl and the substituted phenyl are each independently substituted with one or more (e.g., 2, 3, 4, 5) hydroxyl-C₁₋₆ alkylene groups.

In some embodiments of the first aspect of the present invention, R is selected from the group consisting of heteroaryl, substituted aryl and cycloalkyl, wherein the substituted aryl is substituted with one or more (e.g., 2, 3, 4, 5) hydroxyalkylene groups.

In some embodiments of the first aspect of the present invention, R is selected from the group consisting of 5- to 8-membered (e.g., 6-membered, 7-membered) heteroaryl, substituted 5- to 8-membered (e.g., 6-membered, 7-membered) aryl, and 3- to 8-membered (e.g., 4-membered, 5-membered, 6-membered, 7-membered) cycloalkyl, wherein the substituted 5- to 8-membered (e.g., 6-membered, 7-membered) aryl is substituted with one or more hydroxyl-Ci-io alkylene groups.

In some embodiments of the first aspect of the present invention, R is selected from the group consisting of 5- to 8-membered (e.g. 6-membered, 7-membered) nitrogen-containing heteroaryl, substituted 5- to 8-membered (e.g. 6-membered, 7-membered) aryl and 3- to 8-membered (e.g., 4-membered, 5-membered, 6-membered, 7-membered) cycloalkyl, wherein the substituted 5- to 8-membered (e.g. 6-membered, 7-membered) aryl is substituted with one or more hydroxyalkylene groups.

In some embodiments of the first aspect of the present invention, R is selected from the group consisting of 6-membered nitrogen-containing heteroaryl, substituted phenyl, and 3-to 6-membered (e.g., 4-membered, 5-membered) cycloalkyl, wherein the substituted phenyl is substituted with one or more hydroxyl-C₁₋₆ alkylene groups.

In some embodiments of the first aspect of the present invention, R is selected from the group consisting of pyridyl, substituted phenyl, and 3- to 6-membered (e.g., 4-membered, 5-membered) cycloalkyl, wherein the substituted phenyl is substituted by one or more hydroxyl-C₁₋₄ alkylene groups.

In some embodiments of the first aspect of the present invention, R is selected from the group consisting of pyridin-1-yl, pyridin-2-yl, pyridin-3-yl, pyridin-4-yl, p-hydroxymethylphenyl, o-hydroxymethylphenyl, m-hydroxymethylphenyl, p-hydroxyethylphenyl, o-hydroxyethylphenyl, m-hydroxyethylphenyl, p-hydroxypropylphenyl, cyclopropyl and cyclobutyl.

In some embodiments of the first aspect of the present invention, R is selected from the group consisting of 5- to 8-membered (e.g., 6-membered, 7-membered) nitrogen-containing heteroaryl, 5- to 8-membered (e.g. 6-membered, 7-membered) aryl para-substituted by hydroxyl-C₁₋₆ alkylene group, and 3- to 8-membered (e.g., 4-membered, 5-membered, 6-membered, 7-membered) cycloalkyl.

In some embodiments of the first aspect of the present invention, R is selected from the group consisting of 6-membered nitrogen-containing heteroaryl, 5- to 8-membered (e.g., 6-membered, 7-membered) aryl para-substituted by hydroxyl-C₁₋₆ alkylene group, and 3 - to 8-membered (e.g., 4-membered, 5-membered, 6-membered, 7-membered) cycloalkyl.

In some embodiments of the first aspect of the present invention, R is selected from the group consisting of pyridyl, phenyl para-substituted by hydroxyl-C₁₋₆ alkylene group, and 3- to 8-membered (e.g., 4-membered, 5-membered, 6-membered, 7-membered) cycloalkyl.

In some embodiments of the first aspect of the present invention, R is selected from the group consisting of pyridyl, 5- to 8-membered (e.g., 6-membered, 7-membered) aryl para-substituted by hydroxymethylene, and 3- to 8-membered (e.g. 4-membered, 5-membered, 6-membered, 7-membered) cycloalkyl.

In some embodiments of the first aspect of the present invention, R is selected from the group consisting of

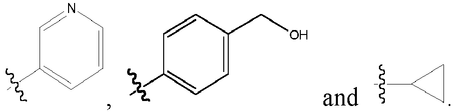

In some embodiments of the first aspect of the present invention, R is selected from the group consisting of substituted aryl, and cycloalkyl, wherein the substituted aryl is substituted with one or more hydroxyalkylene groups.

In some embodiments of the first aspect of the present invention, R is selected from the group consisting of substituted 5- to 8-membered (e.g., 6-membered, 7-membered) aryl, 3-to 8-membered (e.g., 4-membered, 5-membered, 6-membered, 7-membered) cycloalkyl, wherein the substituted 5- to 8-membered (e.g., 6-membered, 7-membered) aryl is substituted with one or more hydroxyalkylene groups.

In some embodiments of the first aspect of the present invention, R is selected from the group consisting of substituted phenyl, 3- to 6-membered (e.g., 4-membered, 5-membered) cycloalkyl, wherein the substituted phenyl is substituted with one or more hydroxyl-C₁₋₆ alkylene groups.

In some embodiments of the first aspect of the present invention, R is selected from the group consisting of p-hydroxymethylphenyl, o-hydroxymethylphenyl, m-hydroxymethylphenyl, p-hydroxyethylphenyl, o-hydroxyethylphenyl, m-hydroxyethylphenyl, p-hydroxypropylphenyl, cyclopropyl and cyclobutyl.

In some embodiments of the first aspect of the present invention, R is selected from the group consisting of aryl para-substituted by hydroxyalkylene group, and cycloalkyl.

In some embodiments of the first aspect of the present invention, R is selected from the group consisting of 5- to 8-membered (e.g. 6-membered, 7-membered) aryl para-substituted by hydroxyl-C₁₋₆ alkylene group and 3- to 6-membered (e.g. 4-membered, 5-membered) cycloalkyl.

In some embodiments of the first aspect of the present invention, R is selected from the group consisting of phenyl para-substituted by hydroxyl-C₁₋₆ alkylene group, and 3- to 6-membered (e.g., 4-membered, 5-membered) cycloalkyl.

In some embodiments of the first aspect of the present invention, R is selected from the group consisting of 5- to 8-membered (e.g., 6-membered, 7-membered) aryl para-substituted by hydroxymethylene, and 3- to 6-membered (e.g., 4-membered, 5-membered) cycloalkyl.

In some embodiments of the first aspect of the present invention, R is selected from

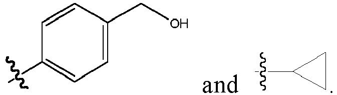

In some embodiments of the first aspect of the present invention, R is aryl substituted by one or more (e.g., 2, 3, 4, 5) hydroxyalkylene groups.

In some embodiments of the first aspect of the present invention, R is 5- to 8-membered (e.g., 6-membered, 7-membered) aryl substituted by one or more (e.g., 2, 3, 4, 5) hydroxyl-C₁₋₆ alkylene groups.

In some embodiments of the first aspect of the present invention, R is phenyl substituted by one or more (e.g., 2, 3, 4, 5) hydroxyl-C₁₋₆ alkylene groups.

In some embodiments of the first aspect of the present invention, R is selected from the group consisting of p-hydroxymethylphenyl, o-hydroxymethylphenyl, m-hydroxymethylphenyl, p-hydroxyethylphenyl, o-hydroxyethylphenyl, m-hydroxyethylphenyl and p-hydroxypropylphenyl.

In some embodiments of the first aspect of the present invention, R is aryl para-substituted by hydroxyalkylene group.

In some embodiments of the first aspect of the present invention, R is 5- to 8-membered (e.g., 6-membered, 7-membered) aryl para-substituted by hydroxyl-C₁₋₆ alkylene group.

In some embodiments of the first aspect of the present invention, R is phenyl para-substituted by hydroxyl-C₁₋₆ alkylene group.

In some embodiments of the first aspect of the present invention, R is 5- to 8-membered (e.g., 6-membered, 7-membered) aryl para-substituted by hydroxymethylene.

In some embodiments of the first aspect of the present invention, R is

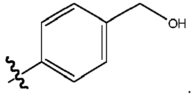

In some embodiments of the first aspect of the present invention, R is cycloalkyl.

In some embodiments of the first aspect of the present invention, R is 3- to 6-membered (e.g., 4-membered, 5-membered) cycloalkyl.

In some embodiments of the first aspect of the present invention, R is

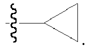

In some embodiments of the first aspect of the present invention, it is selected from the following compounds, their pharmaceutically acceptable salts, their stereoisomers, their pharmaceutically acceptable hydrates or solvates, and their pharmaceutically acceptable esters: 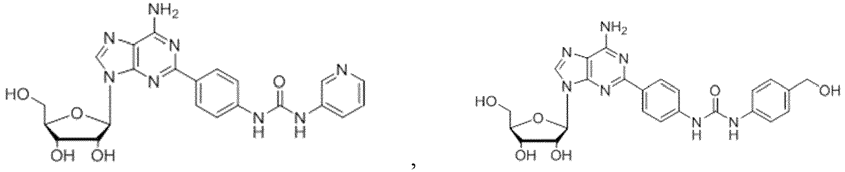 and

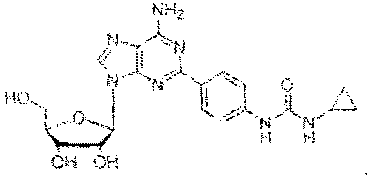

A second aspect of the present invention relates to a method for preparing the compound, its pharmaceutically acceptable salt, its stereoisomer, its pharmaceutically acceptable hydrate or solvate, or its pharmaceutically acceptable ester as described in the first aspect of the present invention, in which the compound represented by Formula I of the present invention is synthesized according to the synthetic scheme as shown below:

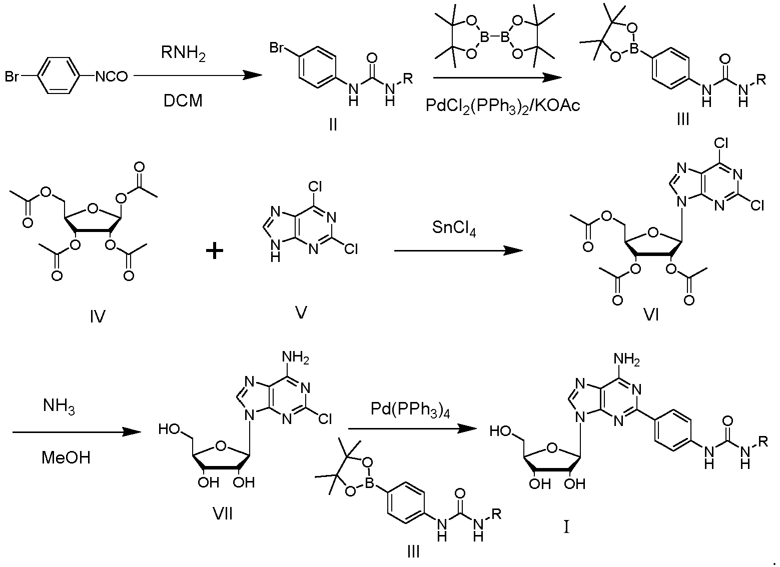

In some embodiments of the second aspect of the present invention, the method comprises the following steps:
(1) reacting 4-bromophenyl isocyanate as starting material with RNH₂ at room temperature in dichloromethane to produce a compound (1-(4-bromophenyl)-3-(R group)urea) represented by Formula II;
(2) adding the compound represented by Formula II, bis(pinacolato)diboron, PdCl₂(PPh₃)₂, potassium acetate into DMF, reacting under nitrogen protection at 70°C to 80°C for 4 hours, and purifying the product to obtain a compound (1-(R group)-3-(4-(4,4,5,5-tetramethyl-1,3,2-dioxaborolan-2-yl)phenyl)urea) represented by Formula III;
(3) performing a substitution reaction of ribofuranose tetraacetate as starting material and 2,6-dichloropurine in the presence of tin tetrachloride as catalyst at 110°C to 130°C to produce a compound (2,6-dichloro-2',3',5'-triacetylpurine nucleoside) represented by Formula VI;
(4) performing a sealed ammonolysis of the compound represented by Formula VI in methanol containing ammonia at 90°C to 110°C to obtain a compound (2-chloroadenosine) represented by Formula VII;
(5) performing a suzuki reaction of the compound represented by Formula VII and the compound represented by Formula III at 100°C, and purifying the product to obtain a compound represented by Formula I;
   in the above Formulas, the definition of R is the same as that in any one of the compound, its pharmaceutically acceptable salt, its stereoisomer, its pharmaceutically acceptable hydrate or solvate, and its pharmaceutically acceptable ester as described in the first aspect of the present invention.

In some embodiments of the second aspect of the present invention, it further comprises: reacting the compound represented by Formula I with an acid to convert it into a pharmaceutically acceptable salt.

A third aspect of the present invention relates to a pharmaceutical composition comprising the compound, its pharmaceutically acceptable salt, its stereoisomer, its pharmaceutically acceptable hydrate or solvate, or its pharmaceutically acceptable ester as described in the first aspect of the present invention, and optionally a pharmaceutical excipient.

In some embodiments of the third aspect of the present invention, the pharmaceutical composition is an A_{2A} adenosine receptor agonist.

In some embodiments of the third aspect of the present invention, the pharmaceutical excipient, according to its source, may be selected from the group consisting of natural material, semi-synthetic material and fully-synthetic material.

In some embodiments of the third aspect of the present invention, the pharmaceutical excipient, according to its function, is selected from the group consisting of solvent, propellant, solubilizer, cosolvent, emulsifier, colorant, binder, disintegrant, filler, lubricant, wetting agent, osmotic pressure regulator, stabilizer, glidant, flavoring agent, preservative, suspending agent, coating material, aromatic, anti-adhesive agent, antioxidant, chelating agent, penetration enhancer, pH-regulator, buffer, plasticizer, surfactant, foaming agent, defoamer, thickener, inclusion agent, humectant, absorbent, diluent, flocculant and deflocculant, filter aid, release retardant.

In some embodiments of the third aspect of the present invention, its administration routes can be divided into oral, injection, mucosal, transdermal or topical administration, nasal or oral inhalation administration, and ocular administration, etc., and the same pharmaceutical excipient may be used in pharmaceutical preparations for different administration routes.

When administered orally, the pharmaceutical composition can be made into any orally acceptable preparation form, including but not limited to tablet, capsule, granule, pill, syrup, oral solution, oral suspension and oral emulsion, etc.. Among them, the carrier used in tablet generally includes lactose and corn starch, and lubricant such as magnesium stearate can also be added. Diluent used in capsule generally includes lactose and dried corn starch. In oral suspension, the active ingredient is usually used by mixing suitable emulsifier and suspending agent. Optionally, some sweetener, aromatic or colorant can be added into the above oral preparation forms.

When administered transdermally or topically, the pharmaceutical composition can be made into an appropriate ointment, lotion or liniment form, in which the active ingredient is suspended or dissolved in one or more carriers. Carriers that can be used in ointment include, but are not limited to: mineral oil, liquid vaseline, white vaseline, propylene glycol, polyoxyethylene, polyoxypropylene, emulsified wax, and water; carriers that can be used in lotion or liniment include, but are not limited to: mineral oil, dehydrated sorbitan monostearate, Tween-60, cetyl ester wax, hexadecenyl aromatic alcohol, 2-octyldodecanol, benzyl alcohol and water.

The pharmaceutical composition can also be administered in the form of injection, including solution for injection, sterile powder for injection and concentrated solution for injection. Among them, usable carriers and solvents include water, Ringer's solution and isotonic sodium chloride solution. In addition, sterilized non-volatile oil, such as monoglyceride or diglyceride, can also be used as solvent or suspension media.

A fourth aspect of the present invention relates to a method for agonizing A_{2A} adenosine receptor (in vivo or in vitro), which comprises applying an effective amount of the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention to A_{2A} adenosine receptor.

In some embodiments of the fourth aspect of the present invention, the A_{2A} adenosine receptor is a mammalian A_{2A} adenosine receptor, preferably a human A_{2A} adenosine receptor.

A fifth aspect of the present invention relates to a method for improving the permeability of blood-brain barrier (in vivo or in vitro), which comprises administering an effective amount of the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention to a subject.

In some embodiments of the fifth aspect of the present invention, the subject is a mammal (e.g., a human) or an in vitro blood-brain barrier model.

A sixth aspect of the present invention relates to a method for promoting the delivery of a drug across blood-brain barrier (in vivo or in vitro), which comprises improving the permeability of blood-brain barrier by the method of the fifth aspect of the present invention, and then administering the drug to the subject; or, administering the drug together with an effective amount of the compound, its pharmaceutically acceptable salt, its stereoisomer, its pharmaceutically acceptable hydrate or solvate, or its pharmaceutically acceptable ester according to the first aspect of the present invention, or the drug together with an effective amount of the pharmaceutical composition according to the third aspect of the present invention to the subject.

In some embodiments of the sixth aspect of the present invention, the subject is a mammal (e.g., a human) or an in vitro blood-brain barrier model.

In some embodiments of the sixth aspect of the present invention, the drug is selected from the group consisting of drug for preventing or treating central nervous system disease, antidote for neurotoxin, and drug for preventing or treating glioma.

A seventh aspect of the present invention relates to a method for preventing or treating a disease associated with A_{2A} adenosine receptor agonistic activity, which comprises providing an effective amount of the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention to a subject in need.

In some embodiments of the seventh aspect of the present invention, the disease associated with A_{2A} adenosine receptor agonistic activity is selected from the group consisting of autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

In some embodiments of the seventh aspect of the present invention, the prevention or treatment of disease associated with A_{2A} adenosine receptor agonistic activity requires to agonize the activity of A_{2A} adenosine receptor.

An eighth aspect of the present invention relates to use of the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention in the manufacture of an A_{2A} adenosine receptor agonist or a medicament for improving the permeability of blood-brain barrier.

A ninth aspect of the present invention relates to use of the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention in the manufacture of a medicament for preventing or treating a disease associated with A_{2A} adenosine receptor agonistic activity.

In some embodiments of the ninth aspect of the present invention, the prevention or treatment of disease associated with A_{2A} adenosine receptor agonistic activity requires to agonize the activity of A_{2A} adenosine receptor.

In some embodiments of the ninth aspect of the present invention, the disease associated with A_{2A} adenosine receptor agonistic activity is selected from the group consisting of autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

A tenth aspect of the present invention relates to use of the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention in the manufacture of a medicament for diagnosing a myocardial perfusion abnormality disease or in the manufacture of a vasodilator (e.g., coronary vasodilator).

An eleventh aspect of the present invention relates to a method for diagnosing a myocardial perfusion abnormality disease, which comprises administering a diagnostically effective amount of the compound, its pharmaceutically acceptable salt, its stereoisomer, its pharmaceutically acceptable hydrate or solvate, or its pharmaceutically acceptable ester according to the first aspect of the present invention, or a diagnostically effective amount of the pharmaceutical composition according to the third aspect of the present invention to a subject in need;
preferably, the subject is a mammal, such as a human.

Another aspect of the present invention relates to the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention, for use in agonizing A_{2A} adenosine receptor, improving the permeability of blood-brain barrier, or promoting the delivery of drug across blood-brain barrier;
preferably, the A_{2A} adenosine receptor is a mammalian A_{2A} adenosine receptor, more preferably a human A_{2A} adenosine receptor;
preferably, the blood-brain barrier is a mammalian (e.g., human) blood-brain barrier or an in vitro blood-brain barrier model;
preferably, the drug is selected from the group consisting of drug for preventing or treating central nervous system disease, antidote for neurotoxin, and drug for preventing or treating glioma.

Another aspect of the present invention relates to the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention, for use in preventing or treating a disease associated with A_{2A} adenosine receptor agonistic activity;
preferably, the disease associated with A_{2A} adenosine receptor agonistic activity is selected from the group consisting of autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

In some embodiments of the present invention, the prevention or treatment of disease associated with A_{2A} adenosine receptor agonistic activity requires to agonize the activity of A_{2A} adenosine receptor.

Another aspect of the present invention relates to the compound, its pharmaceutically acceptable salt, its stereoisomer, its pharmaceutically acceptable hydrate or solvate, or its pharmaceutically acceptable ester according to the first aspect of the present invention, or the pharmaceutical composition according to the third aspect of the present invention, for use in diagnosing a myocardial perfusion abnormality disease or dilating a blood vessel (e.g., a coronary artery).

Another aspect of the present invention relates to a pharmaceutical composition, which comprises the compound, its pharmaceutically acceptable salt, its stereoisomer, its pharmaceutically acceptable hydrate or solvate, or its pharmaceutically acceptable ester according to the first aspect of the present invention, and a drug suitable for delivery across blood-brain barrier and optionally a pharmaceutical excipient.

In some embodiments of the present invention, the drug suitable for delivery across blood-brain barrier is selected from the group consisting of drug for preventing or treating central nervous system disease, antidote for neurotoxin, and drug for preventing or treating glioma.

In some embodiments of the present invention, the pharmaceutical excipient, according to its source, may be selected from the group consisting of natural product, semi-synthetic product and fully-synthetic product.

In some embodiments of the present invention, the pharmaceutical excipient, according to its function, is selected from the group consisting of solvent, propellant, solubilizer, cosolvent, emulsifier, colorant, binder, disintegrant, filler, lubricant, wetting agent, osmotic pressure regulator, stabilizer, glidant, flavoring agent, preservative, suspending agent, coating material, aromatic, anti-adhesive agent, antioxidant, chelating agent, penetration enhancer, pH-regulator, buffer, plasticizer, surfactant, foaming agent, defoamer, thickener, inclusion agent, humectant, absorbent, diluent, flocculant and deflocculant, filter aid, release retardant.

In some embodiments of the present invention, its administration routes can be divided into oral, injection, mucosal, transdermal or topical administration, nasal or oral inhalation administration, and ocular administration, etc., and the same pharmaceutical excipient may be used in pharmaceutical preparations for different administration routes.

In the present invention, if there is no other description, wherein:
The term "pharmaceutically acceptable salt" refers to a salt formed by an acidic functional group (e.g., -COOH, -OH, etc.) in the compound with an appropriate inorganic or organic cation, and a salt formed by a basic functional group (e.g., -NH₂, etc.) in the compound with an appropriate inorganic or organic anion. "Pharmaceutically acceptable salt" includes, but is not limited to: alkali metal salt, such as sodium salt, potassium salt, lithium salt, etc.; alkaline earth metal salt, such as calcium salt, magnesium salt, etc.; other metal salt, such as aluminum salt, iron salt, zinc salt, copper salt, nickel salt, cobalt salt, etc.; inorganic alkali salt, such as ammonium salt; organic alkali salt, such as tert-octylamine salt, dibenzylamine salt, morpholinium salt, glucosamine salt, alkyl phenylglycinate salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt, tris(hydroxymethyl)aminomethane salt; hydrohalide salt, such as hydrofluoride, hydrochloride, hydrobromide, hydroiodide, etc.; inorganic acid salt, such as nitrate, perchlorate, sulfate, phosphate, etc.; lower alkanesulfonate, such as methanesulfonate, trifluoromethanesulfonate, ethanesulfonate, etc.; arylsulfonate, such as benzenesulfonate, p-benzenesulfonate, etc.; organic acid salt, such as acetate, malate, fumarate, succinate, citrate, tartrate, oxalate, maleate, etc.; amino acid salt, such as glycinate, trimethylglycinate, argininate, ornithinate, glutamate, aspartate, etc.

The term "stereoisomer" includes conformational isomer and configurational isomer, wherein the configurational isomer mainly includes cis/trans isomer and optical isomer. The compound of the present invention may exist in the form of stereoisomer, and therefore encompass all possible stereoisomer forms, and any combination or any mixture thereof.

According to certain embodiments of the present invention, the compound of general Formula (I) of the present invention can form a pharmaceutically acceptable ester with an organic or inorganic acid, and the pharmaceutically acceptable ester includes phosphate, sulfate, nitrate, formate, acetate, propionate, butyrate, valerate, caproate and other esters that are hydrolyzable in vivo.

The carrier of the present invention includes, but is not limited to: ion exchanger, alumina, aluminum stearate, lecithin, serum protein such as human albumin, buffer substance such as phosphate, glycerin, sorbic acid, potassium sorbate, partial glyceride mixture of saturated plant fatty acid, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin.

The term "excipient" as used in the present invention refers to an additive other than the main drug in a pharmaceutical preparation. It is stable in nature, has no incompatibility with the main drug, does not produce side effects, does not affect the therapeutic effect, is not prone to deform, dry, crack, mildew and moth. It is harmless to the human body, has no physiological effect, does not produce chemical or physical effects with the main drug, and does not affect the content determination of the main drug, etc. For example, binder, filler, disintegrant, lubricant in tablet, and preservative, antioxidant, flavor, aromatic, cosolvent, emulsifier, solubilizer and osmotic pressure regulator, colorant. in oral liquid preparation can all be called excipients.

The term "pharmaceutical excipient" refers to a substance, in addition to the active ingredient, that is contained in pharmaceutical preparation and has been reasonably evaluated in terms of safety during drug production and prescription formulation. In addition to shaping, acting as carrier, and improving stability, pharmaceutical excipient also has important functions such as solubilization, hydrotropy, sustained- and controlled-release.

The term "effective amount" refers to an amount that is sufficient to treat or prevent or diagnose a patient's disease but is low enough to avoid serious side effects (at a reasonable benefit/risk ratio) within the scope of reasonable medical judgment. The therapeutically or prophylactically or diagnostically effective dose of the compound will change according to the specific compound selected (e.g., considering the potency, effectiveness and half-life of the compound), the administration route selected, the disease to be treated or prevented or diagnosed, the severity of the disease to be treated or prevented or diagnosed, the age, size, weight, and physical illness of the patient being treated, the medical history of the patient being treated, the duration of treatment or prevention or diagnosis, the nature of concurrent therapy, the effects of treatment or prevention or diagnosis required and other factors, but they can still be routinely determined by those skilled in the art.

In addition, it should be pointed out that the specific dosage and usage of the compound of the general Formula (I) described in the present application for different patients depend on many factors, including the patient's age, weight, gender, natural health status, nutritional status, and the compound's activity strength, administration time, metabolic rate, the severity of disease and the subjective judgment of physician. The preferred dosage used herein is between 0.001 and 100 mg/kg body weight/day.

The term "heteroaryl" refers to a monovalent group formed by an aromatic monocyclic or polycyclic compound containing at least one N, O or S heteroatom, such as 5- to 8-membered (e.g., 5-membered, 6-membered, 7-membered, 8-membered) nitrogen-containing heteroaryl, 5- to 8-membered (e.g., 5-membered, 6-membered, 7-membered, 8-membered) oxygen-containing heteroaryl, 5- to 8-membered (e.g., 5-membered, 6-membered, 7-membered, 8-membered) sulfur-containing heteroaryl. Specific examples of nitrogen-containing heteroaryl group include, but are not limited to, pyrazolyl, pyrrolyl, isoxazolyl, isothiazolyl, thiazolyl, pyridyl, imidazolyl, quinolinyl, and pteridinyl.

The term "aryl" refers to a monovalent group formed by a monocyclic or polycyclic compound with aromaticity, such as 5- to 8-membered aryl, phenyl, 7-membered aryl, and the like.

The term "cycloalkyl" refers to a monocyclic saturated alkyl, for example, 3- to 8-membered cycloalkyl contains 3 to 8 ring members, such as 3-membered, 4-membered, 5-membered, 6-membered, 7-membered, 8-membered cycloalkyl. Specific examples include, but are not limited to: cyclopropyl, cyclobutyl, cyclopentyl, cyclohexyl and the like.

The term "alkyl" refers to a straight or branched chain alkyl having multiple carbon atoms, such as C₁₋₁₀ alkyl, C₁₋₆ alkyl, C₁₋₄ alkyl, C₁₋₂ alkyl, C₁ alkyl, C₂ alkyl, C₃ alkyl, C₄ alkyl, C₅ alkyl. Specific examples include, but are not limited to, methyl, ethyl, propyl, isopropyl, n-butyl, isobutyl, sec-butyl, tert-butyl, n-pentyl, n-hexyl and the like.

The term "alkylene" refers to a divalent group formed by removing one hydrogen atom from an alkyl, wherein the definition of "alkyl" is as described above. Examples include C₁₋₁₀ alkylene, C₁₋₆ alkylene, C₁₋₄ alkylene, C₁₋₂ alkylene, C₁ alkylene, C₂ alkylene, C₃ alkylene, C₄ alkylene, C₅ alkylene, C₆ alkylene.

The term "hydroxyalkylene" refers to -(CH₂)ₙ-OH, such as hydroxyl-C₁₋₁₀ alkylene, hydroxyl-C₁₋₆ alkylene, hydroxyl-C₁₋₄ alkylene, hydroxyl-C₁₋₂ alkylene, hydroxymethylene, hydroxyethylene, hydroxypropylene, etc.

The term "pyridin-1-yl" has a structural formula of

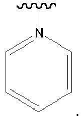

The term "pyridin-2-yl" has a structural formula of

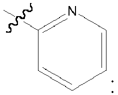

The term "pyridin-4-yl" has a structural formula of

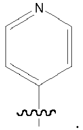

The term "p-hydroxymethylphenyl" has a structural formula of

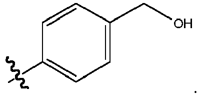

The term "o-hydroxymethylphenyl" has a structural formula of

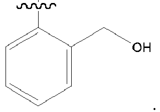

The term "m-hydroxymethylphenyl" has a structural formula of

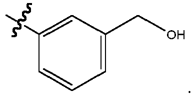

The beneficial effects achieved by the present invention:
1. The adenosine compound, its pharmaceutically acceptable salt, its stereoisomer, its pharmaceutically acceptable hydrate or solvate, or its pharmaceutically acceptable ester according to the present invention has an agonistic activity on A_{2A} adenosine receptor.
2. The adenosine compound, its pharmaceutically acceptable salt, its stereoisomer, its pharmaceutically acceptable hydrate or solvate, or its pharmaceutically acceptable ester according to the present invention can improve the permeability of blood-brain barrier, thereby facilitating the delivery of drug across the blood-brain barrier.
3. The adenosine compound, its pharmaceutically acceptable salt, its stereoisomer, its pharmaceutically acceptable hydrate or solvate, or its pharmaceutically acceptable ester according to the present invention can prevent or treat a disease associated with A_{2A} adenosine receptor agonistic activity.

### Specific Models for Carrying Out the present invention

The embodiments of the present invention will be clearly and completely described below in conjunction with examples. Obviously, the described examples are only a part of the examples of the present invention, rather than all of the examples. The following description of at least one exemplary embodiment is actually only illustrative, and in no way serves as any limitation to the present invention and its application or use. Based on the embodiments of the present invention, all other embodiments obtained by those of ordinary skill in the art without creative work shall fall within the protection scope of the present invention.

Unless otherwise stated: all temperatures were expressed in °C (degrees Celsius); the structure of the compound was determined by nuclear magnetic resonance (NMR) or mass spectrometry (MS); the melting point m.p. of the compound was determined by RY-1 melting point instrument, and the thermometer had not been corrected. The m.p. was given in °C; 1H NMR was measured by JEOL JNM-ECA-400 nuclear magnetic resonance instrument; mass spectrum was measured by API3000 (ESI) instrument; all reaction solvents that were not specified were subject to standardized pretreatment.

### Example 1: Synthesis of 1-{4-{6-amino-9-[(2R,3R,4S,5R)-3,4-dihydroxy-5-(hydroxymethyl)tetrahydrofuran-2-yl]-9H-purin-2-yl}phenyl}-3-(pyridin-3-yl)urea (Compound 1)

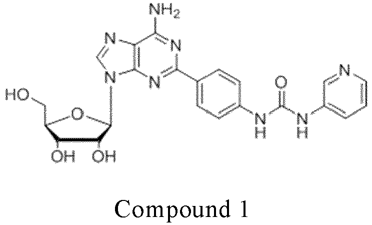

Compound 1 was prepared according to the following reaction scheme, wherein R was pyridin-3-yl.

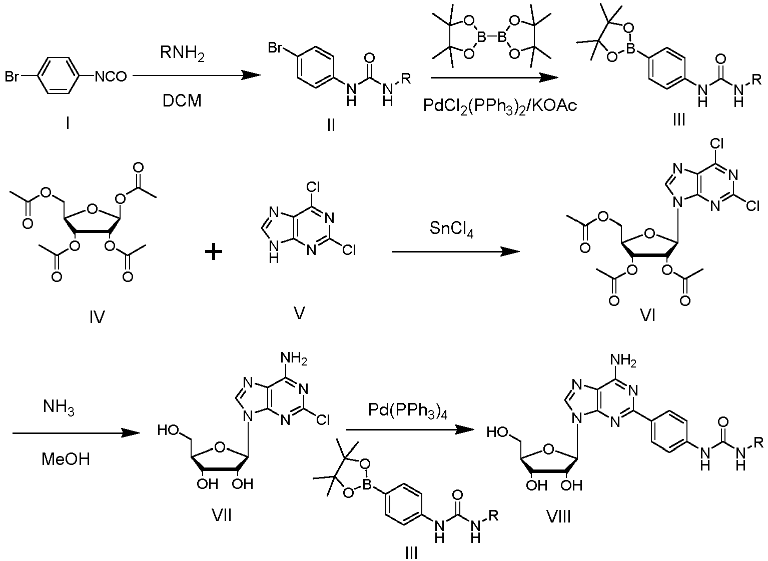

### (1) Synthesis of 1-(4-bromophenyl)-3-(pyridin-3-yl) urea (II)

0.6g (3.03mmol) of 4-bromophenylisocyanate was added to 100ml of dichloromethane and stirred for dissolution, 10ml of dichloromethane solution containing 0.34g (3.63mmol) of 3-aminopyridine was gradually added dropwise, after the formation of precipitate, the reaction was continued for 2 hours, and filtration was carried out to obtain 0.85 g of white solid 1-(4-bromophenyl)-3-(pyridin-3-yl)urea, which was directly used in the next reaction.

### (2) Synthesis of 1-(pyridin-3-yl)-3-[4-(4,4,5,5-tetramethyl-1,3,2-dioxaborolan-2-yl)phenyl]urea (III)

0.75g (2.56mmol) of 1-(4-bromophenyl)-3-(pyridin-3-yl)urea, 1.3g (5.1mmol) of bis(pinacolato)diboron, 0.9g (1.78mmol) of PdCl₂ (PPh₃)₂, 0.7g (7.7mmol) of potassium acetate were added to 50ml of DMF, and reacted at 80°C for 4 hours under nitrogen protection. After the reaction, it was extracted with dichloromethane/water, the organic layer was collected and washed with water and saturated brine, dried with anhydrous Na₂SO₄, filtrated, and purified by column chromatography to obtain 0.7g of white solid 1-(R group)-3-[4-(4,4,5,5-tetramethyl-1,3,2-dioxaborolan-2-yl)phenyl]urea, which was directly used in the next reaction.

### (3) Synthesis of (2R,3R,4R,5R)-2-(acetoxymethyl)-5-(2,6-dichloro-9H-purin-9-yl) tetrahydrofuran-3,4-diyl diacetate (VI)

21g (0.066mol) of (2S,3R,4R,5R)-5-(acetoxymethyl)tetrahydrofuran-2,3,4-triethyl triacetate (IV) was heated to 90°C until it became clear, and at this time 12g (0.063mol) of 2,6-dichloropurine (V) and 0.3g of tin tetrachloride were added and stirred, and then heated to 120°C and stirred for 15 minutes. The solvent was evaporated in vacuo and the residue was cooled. Methanol (50 ml) was added to the residue, and the crude solid product was separated by filtration. The crude product was recrystallized in ethanol to obtain 12g of pale yellow powder product (2R,3R,4R,5R)-2-(acetoxymethyl)-5-(2,6-dichloro-9H-purin-9-yl) tetrahydrofuran-3,4-diyl diacetate, which was directly used in the next reaction.

### (4) Synthesis of (2R,3R,4S,5R)-2-(6-amino-2-chloro-9H-purin-9-yl)-5-(hydroxymethyl) tetrahydrofuran-3,4-diol (VII)

10g (0.022mol) of (2R,3R,4R,5R)-2-(acetoxymethyl)-5-(2,6-dichloro-9H-purin-9-yl) tetrahydrofuran-3,4-diyl diacetate (VI) was heated to 100°C in 200ml of methanol solution containing ammonia and kept in an autoclave for 24 hours. The solution was further stirred for 24 hours until room temperature, and then the solution was evaporated to dryness under reduced pressure to remove ammonia. The residue was purified by flash chromatography, in which the mixed solvent of CHCl₃ and MeOH was used as the eluent. The product was dried below 50°C to obtain 4.5g of light yellow powder (2R,3R,4S,5R)-2-(6-amino-2-chloro-9H-purin-9-yl)-5-(hydroxymethyl)tetrahydrofuran-3,4-diol, which was directly used in the next reaction.

### (5) Synthesis of 1-{4-{6-amino-9-[(2R,3R,4S,5R)-3,4-dihydroxy-5-(hydroxymethyl) tetrahydrofuran-2-yl]-9H-purin-2-yl}phenyl}-3-(pyridin-3-yl)urea (VIII, i.e., Compound 1)

0.5g (0.0017mol) of (2R,3R,4S,5R)-2-(6-amino-2-chloro-9H-purin-9-yl)-5-(hydroxymethyl)tetrahydrofuran-3,4-diol (VII) was added to 0.6g (0.0018mol) of 1-(pyridin-3-yl)-3-[4-(4,4,5,5-tetramethyl-1,3,2-dioxaborolan-2-yl)phenyl]urea (III, 1.1 equivalent), then 3ml of ethylene glycol dimethyl ether, 10ml of methanol, 5ml of 2M Na₂CO₃ aqueous solution were added and stirred, 1.0g (0.0009mol) of Pd(PPh₃)₄ (0.5 equivalent) was added and then the air in the reaction flask was evacuated by introducing nitrogen gas, it was heated to 100°C and refluxed overnight. Then the heating was stopped and it was cooled to room temperature, 300ml of methanol was added and stirred for dissolution, after filtration a crude product was separated by silica gel column chromatography from the filtrate mixture . The crude product was further purified by medium pressure preparative chromatography using C18 reverse phase column to obtain 116 mg of white solid (Compound 1). m.p. 230°C; 1H NMR (DMSO-d6): δ (ppm) 9.05(s, 1H), 8.94(s, 1H), 8.63(s, 1H), 8.36(s, 1H), 8.29(d, 2H, J=8.0Hz), 8.21(d, 2H, J=4.8Hz), 7.98(d, 1H, J=9.6Hz), 7.57(d, 2H, J=8.0Hz), 7.35-7.32(m, 2H), 5.99(d, 1H, J=5.2Hz), 5.51(s, 1H), 5.27(s, 1H), 5.07(s, 1H), 4.75(s, 1H), 4.23(s, 1H), 3.97(s, 1H), 3.72-3.57(m, 2H); HRMS (ESI+) m/z [M + H]+ calculated for C₂₂H₂₂N₈O₅: 479.1786; found: 479.1786.

Compounds 2 to 3 as follows were prepared with reference to the method of Example 1, in which step (1) and step (2) were used to synthesize different 1-(R group)-3-[4-(4,4,5,5-tetramethyl-1,3,2-dioxaborolan-2-yl)phenyl]urea (III) that was used to replace the side chain compound (III) in step (5) for preparation.

### Example 2: Synthesis of 1-{4-{6-amino-9-[(2R,3R,4S,5R)-3,4-dihydroxy-5-(hydroxymethyl)tetrahydrofuran-2-yl]-9H-purin-2-yl}phenyl}-3-[4-(hydroxymethyl)phenyl ]urea (Compound 2)

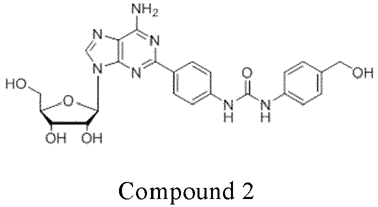

### (1) Synthesis of 1-(4-bromophenyl)-3-[4-(hydroxymethyl)phenyl]urea (II)

By referring to the method of step (1) in Example 1, 3-aminopyridine was replaced with 4-aminobenzyl alcohol, and the remaining operations were the same as those in step (1) of Example 1 to obtain 0.91g of white solid 1-(4-bromophenyl)-3-[4-(hydroxymethyl)phenyl]urea, which was directly used in the next reaction.

### (2) Synthesis of 1-[4-(hydroxymethyl)phenyl]-3-[4-(4,4,5,5-tetramethyl-1,3,2-dioxaborolan-2-yl)phenyl]urea (III)

By referring to the step (2) of Example 1, 1-(4-bromophenyl)- 3-(pyridin-3-yl)urea was replaced with 1-(4-bromophenyl)-3-[4-(hydroxymethyl) phenyl]urea, and the remaining operations were the same as those in step (2) of Example 1 to obtain 0.76g of white solid 1-[4-(hydroxymethyl)phenyl]-3-[4-(4,4,5,5-tetramethyl-1,3,2-dioxaborolan-2-yl)phenyl]urea, which was directly used in the next reaction.

### (3) Synthesis of 1-{4-{6-amino-9-[(2R,3R,4S,5R)-3,4-dihydroxy-5-(hydroxymethyl) tetrahydrofuran-2-yl]-9H-purin-2-yl}phenyl}-3-[4-(hydroxymethyl)phenyl]urea (Compound 2)

By referring to the method of step (5) in Example 1, 1-(pyridin-3-yl)-3-[4-(4,4,5,5-tetramethyl-1,3,2-dioxaborolan-2-yl)phenyl]urea was replaced with 1-[4-(hydroxymethyl)phenyl]-3-[4-(4,4,5,5-tetramethyl-1,3,2-dioxaborolan-2-yl)phenyl]ure a, and the remaining operations were the same as those in step (5) of Example 1 to obtain 138 mg of white solid (Compound 2). m.p. 174°C; 1H NMR (DMSO-d6): δ (ppm) 8.89(s, 1H), 8.73(s, 1H), 8.35(s, 1H), 8.27(d, 2H, J=8.8Hz), 7.55(d, 2H, J=8.4Hz), 7.43(d, 2H, J=8.4Hz), 7.30(s, 2H), 7.24(d, 2H, J=8.8Hz), 5.98(d, 1H, J=6.0Hz), 5.50(d, 1H, J=6.0Hz), 5.26(d, 1H, J=4.8Hz), 5.11-5.05(m,2H), 4.74(dd, 1H, J=5.6Hz, 5.6Hz), 4.43(d, 2H, J=5.6Hz), 4.23(dd, 1H, J=3.6Hz, 4.4Hz), 3.96(d, 1H, J=3.6Hz), 3.73-3.55(m, 2H); HRMS (ESI+) m/z [M + H]+ calculated for C₂₄H₂₅N₇O₆: 508.1939; found: 508.1938.

### Example 3: Synthesis of 1-{4-{6-amino-9-[(2R,3R,4S,5R)-3,4-dihydroxy-5-(hydroxymethyl)tetrahydrofuran-2-yl]-9H-purin-2-yl}phenyl}-3-cyclopropylurea (Compound 3)

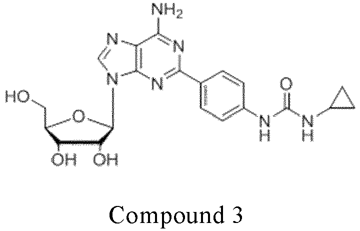

### (1) Synthesis of 1-(4-bromophenyl)-3-cyclopropylurea (II)

By referring to the method of step (1) in Example 1, 3-aminopyridine was replaced with cyclopropylamine, and the remaining operations were the same as those in step (1) of Example 1 to obtain 0.72g of white solid 1-(4-bromophenyl)-3-cyclopropylurea, which was directly used in the next reaction.

### (2) Synthesis of 1-cyclopropyl-3-[4-(4,4,5,5-tetramethyl-1,3,2-dioxaborolan-2-yl)phenyl]urea (III)

By referring to the method of step (2) in Example 1, 1-(4-bromophenyl)-3-(pyridin-3-yl)urea was replaced with 1-(4-bromophenyl)-3-cyclopropylurea, and the remaining operations werethe same as those in step (2) of Example 1 to obtain0.59g of white solid 1-cyclopropyl-3-[4-(4,4,5,5-tetramethyl-1,3,2-dioxaborolan-2-yl)phenyl]urea, which was directly used in the next reaction.

### (3) Synthesis of 1-{4-{6-amino-9-[(2R,3R,4S,5R)-3,4-dihydroxy-5-(hydroxymethyl) tetrahydrofuran-2-yl]-9H-purin-2-yl}phenyl}-3-cyclopropylurea (Compound 3)

By referring to the method of step (5) in Example 1, 1-(pyridin-3-yl)-3-[4-(4,4,5,5-tetramethyl-1,3,2-dioxaborolan-2-yl)phenyl]urea was replaced with 1-cyclopropyl-3-[4-(4,4,5,5-tetramethyl-1,3,2-dioxaborolan-2-yl)phenyl]urea, and the remaining operations were the same as those in step (5) of Example 1to obtain 135 mg of white solid (Compound 3). m.p. 174°C; 1H NMR (DMSO-d6): δ (ppm) 8.51(s, 1H), 8.33(s, 1H),8.21(d, 2H, J=8.4Hz), 7.49(d, 3H, J=8.4Hz), 7.24(s, 2H), 6.47(s, 1H), 5.97(d, 1H, J=6.0Hz), 5.46(d, 1H, J=6.0Hz), 5.22(d, 1H, J=4.4Hz),5.03(t, 1H, J=5.6Hz), 4.72(d, 1H, J=5.6Hz), 4.22(d, 1H, J=4.0Hz), 3.96(d, 1H, J=3.6Hz), 3.71-3.54(m, 2H), 0.64(d, 2H, J=5.2Hz), 0.42(s, 2H); HRMS (ESI+) m/z [M + H]+ calculated for C₂₀H₂₃N₇O₅: 442.1833; found: 442.1833.

### Example 4: Radioligand binding test

1) Experimental materials:
[3H]-CGS21680 (2-[p-(2-carboxyethyl)phenylethylamino]-5'-N-ethylcarboxamido-adenosine, [carboxy-1-ethyl-3H(N)]-; 250µCi): purchased from PerkinElmer Research Products (Boston, MA);
The cell membrane stably transfected with (human) A_{2A} adenosine receptor was prepared in HEK-293 cells, and the cell membrane was obtained from PerkinElmer Research Products (Boston, MA);
CGS 21680 (2-[p-(2-carboxyethyl)phenylethylamino]-5'-N-ethylcarboxamidoadenosine) was purchased from Selleck (Shanghai, CN);
All other reagents were of analytical grade and obtained from commercial sources.

2) Experimental method:
The A_{2A} adenosine receptors used were all expressed in the cell membrane. Each compound was continuously diluted 3 times with DMSO (Solarbio, D8371-250ml) to generate compound source plates with 10 different concentrations (10µM, 3.3µM, 1.1µM, 0.37µM, 0.12µM, 0.0412µM, 0.0137µM, 0.0046µM, 0.0015µM, 0.0005µM), 250 nL of compounds were added to 384-well Opti-plate, sealed with parafilm; 1 mL of assay buffer (containing 50 mM Tris-HCl, pH 7.4, 10 mM MgCl₂, 1 mM EDTA, 1µg/mL adenosine deaminase) was added to 20 U hA_{2A} HEK-293 cell membrane for dilution, 0.75 µCi [3H]-CGS21680 (final 25 nM) was added to the diluted cell membrane and mixed well; 50 µL of the prepared cell membrane dilution was transferred to the 384-well Opti-plate containing fresh compounds and incubated at 25°C for 90 minutes; 100 µL of 0.5% polyethyleneimine solution (PEI) was added to UNIFILTER-96 GF/B filter plate, and soaked at 4°C for 90 minutes, then the UNIFILTER-96 GF/B filter plate was washed twice with 500 µL of washing buffer per well (washing buffer contained 50mM Tris-HCl pH 7.4, 154mM NaCl) which was transferred by Cell Harvester; then, the mixture system in the Opti-plate was transferred to the cleaned UNIFILTER-96 GF/B filter plate, and then the UNIFILTER-96 GF/B filter plate was washed 9 times with 500 µL of washing buffer per well (washing buffer contained 50mM Tris-HCl, pH 7.4, 154mM NaCl), and incubated for 3 minutes in a 37°C incubator; 40 µL of ULTIMA GOLD scintillation fluid (Perkin Elmer, Cat #77-16061) was added to each well, MicroBeta liquid scintillation counter (PerkinElmer) was used to read CPM (count per minute) value. The specific binding percentage of [3H]CGS21680 was calculated according to the CPM value, % specific binding of [3H]CGS21680 = (CPMₛₐₘₚₗₑ - CPM_{Low Control}) / (CPM_{High Control} - CPM_{Low Control}) ^{∗} 100, wherein High Control was 0.5% DMSO, Low Control was 100 µM CGS21680. According to the compound concentration and the specific binding percentage of [3H]CGS21680, curve fitting was performed to calculate IC₅₀ value.
3) Experimental results:
The binding inhibition constant (Ki) value was calculated from the IC₅₀ value according to the Cheng-Prusoff equation, Ki = IC₅₀ / (1+[S]/Km), wherein [S] was radioligand concentration (25nM), and Km was human A_{2A}AR dissociation constant of [3H]CGS21680 (22 nM). Table 1 showed the binding inhibition constant Ki values of Compounds 1 to 3 of the present invention binding to A_{2A} adenosine receptor.

**Table 1: Test results of compounds binding to A_{2A} adenosine receptor**

| Compound | IC₅₀(nM) | Ki (nM) |
|---|---|---|
| Compound 1 | 1662 | 778 |
| Compound 2 | 35.0 | 16.4 |
| Compound 3 | 34.0 | 15.9 |

### Example 5: cAMP test of A_{2A} adenosine receptor

1) Experimental materials:
DMEM/F12, G418, Penicillin-Streptomycin, Versene solution, HEPES, Hank's buffer saline aqueous solution, PBS (pH 7.4, 1×, sterile), FBS, 7.5% BSA stabilizer, Rolipram, NECA, were purchased from Gibico, Hyclone and Sigma, respectively;
LANCE^{®} Ultra cAMP kit (containing Eu-cAMP tracer, Ulight-anti-cAMP reagent, cAMP detection buffer) and hADORA_{2A}-HEK293 cells were purchased from PerkinElmer Research Products (Boston, MA);
All other reagents were of analytical grade and obtained from commercial sources;
384-well polypropylene microplate and 384-well white solid plate were purchased from Labcyte and Corning, respectively.
Experimental instruments: TECAN automated pipetting workstation, Echo ultrasonic pipetting system and EnVison microplate reader were purchased from TECAN, Labcyte and Envision, respectively.

2) Experimental method:
The cells stably expressing human adenosine receptor A_{2A} (hADORA_{2A}-HEK293 cells) were cultured in DMEM/F12 medium containing 10% FBS, 1× Penicillin-Streptomycin and 400 µg/ml G418 in the environment of 37°C and 5% CO₂. Before the experiment, the cells were digested with Versene solution. The cells were collected by centrifugation at 200g and room temperature for 5 minutes using a centrifuge, and finally resuspended using the assay buffer (the assay buffer contained Hank's buffer saline aqueous solution, 1M HEPES, 7.5% BSA stabilizer, pH 7.4, 20mM Rolipram). The compound was continuously diluted 3 times in a 384-well polypropylene microplate with DMSO through TECAN automated pipetting workstation to prepare compound source plates with 11 concentration points (10mM, 3.33mM, 1.11mM, 0.37mM, 0.12mM, 0.041mM, 0.013mM, 4.57×10⁻³mM, 1.52×10⁻³mM, 5×10⁻⁴mM and 1.7×10⁻⁴mM). The test compound was tranfered from the compound source plate to assay plate by Echo ultrasonic pipetting system (Labcyte), and the transfer volume of the compound was 10 nl/well. The hADORA_{2A}-HEK293 cell suspension was diluted to 30,000 cells/ml with assay buffer (the assay buffer contained Hank's buffer saline aqueous solution, 1M HEPES, 20mM Rolipram, 7.5% BSA stabilizer, pH 7.4), and then the cell suspension was transferred to the assay plate in a volume of 10 µl/well (300 cells/well). The assay plate was centrifuged at 150g for 1 minute and pre-incubated for 30 minutes at room temperature. Eu-cAMP tracer working solution (including 40µl of Eu-cAMP tracer, 1.96ml of cAMP detection buffer) (5µl/well) was added to the assay plate, and then Ulight-anti-cAMP working solution (13µl of Ulight-anti-cAMP agent and 1.95ml of cAMP detection buffer) (5µl/well) was added to the assay plate. The assay plate was rotated at 150g for 30 seconds and incubated at room temperature for 30 minutes. EnVison microplate reader (EnVision multimode plate reader, PerkinElmer) was used to determine the level of cyclic adenosine monophosphate in the final solution (λₑₓ= 320nm, λₑₘ= 665nm & 615nm). The EC₅₀ (nM) value that stimulated the production level of cyclic adenosine monophosphate when the test compound interacted with the A_{2A} adenosine receptor was calculated. The A_{2A} receptor agonist titer of the compound was expressed as the EC₅₀ (nM) value that stimulated the production level of cyclic adenosine monophosphate when the compound interacted with the A_{2A} adenosine receptor.
3) Experimental results:
When the test compound interacted with A_{2A}AR, the EC₅₀ (nM) value that stimulated the level of cyclic AMP was shown in Table 2. The results show that the compound of the present invention is shown to be an hA_{2A}AR agonist. It is generally accepted in this field that cAMP EC₅₀ value <10µM indicates significant result, and cAMP EC₅₀ value <1µM indicates a strong agonistic activity.

**Table 2: EC₅₀ value test results of compound for A_{2A} agonist function determination**

| Compound | cAMP EC₅₀ (nM) |
|---|---|
| Compound 2 | 227.7 |

Finally, it should be noted that the above examples are only used to illustrate the technical solution of the present invention but not to limit it; although the present invention has been described in detail with reference to the preferred examples, those of ordinary skill in the art should understand that: the specific implementation of the present invention can be modified or some technical features can be equivalently replaced without departing from the spirit of the technical solution of the present invention, and all of them shall be covered by the scope of the technical solution sought to be protected by the present invention.

## Claims

1. A compound represented by Formula I, its pharmaceutically acceptable salt, its stereoisomer, its pharmaceutically acceptable hydrate or solvate, or its pharmaceutically acceptable ester, 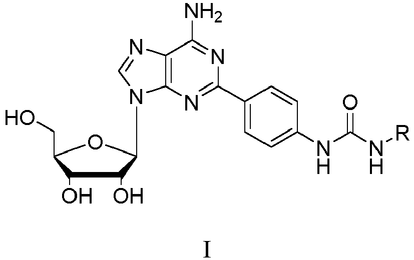
wherein, R is selected from the group consisting of heteroaryl, substituted heteroaryl, aryl, substituted aryl, cycloalkyl, substituted cycloalkyl, alkyl, and substituted alkyl, wherein the substituted heteroaryl, the substituted aryl, the substituted cycloalkyl and the substituted alkyl are each independently substituted with one or more hydroxyalkylene groups;
preferably, R is selected from the group consisting of 5- to 8-membered heteroaryl, substituted 5- to 8-membered heteroaryl, 5- to 8-membered aryl, substituted 5- to 8-membered aryl, 3- to 8-membered cycloalkyl, substituted 3- to 8-membered cycloalkyl, C₁₋₁₀ alkyl, and substituted C₁₋₁₀ alkyl, wherein the substituted 5- to 8-membered heteroaryl, the substituted 5- to 8-membered aryl, the substituted 3- to 8-membered cycloalkyl and the substituted C₁₋₁₀ alkyl are each independently substituted with one or more hydroxyl-Ci-io alkylene groups.

2. The compound, its pharmaceutically acceptable salt, its stereoisomer, its pharmaceutically acceptable hydrate or solvate, or its pharmaceutically acceptable ester according to claim 1, wherein R is selected from the group consisting of heteroaryl, substituted heteroaryl, substituted aryl, and cycloalkyl, wherein the substituted heteroaryl and the substituted aryl are each independently substituted with one or more hydroxyalkylene groups;
preferably, R is selected from the group consisting of 5- to 8-membered heteroaryl, substituted 5- to 8-membered heteroaryl, substituted 5- to 8-membered aryl, and 3- to 8-membered cycloalkyl, wherein the substituted 5- to 8-membered heteroaryl and the substituted 5- to 8-membered aryl are each independently substituted with one or more hydroxyl-Ci-io alkylene groups;
more preferably, R is selected from the group consisting of pyrrolyl, substituted pyrrolyl, imidazolyl, substituted imidazolyl, thiazolyl, substituted thiazolyl, furyl, substituted furyl, pyridyl, substituted pyridyl, substituted phenyl, and 3- to 6-membered cycloalkyl, wherein the substituted pyrrolyl, the substituted imidazolyl, the substituted thiazolyl, the substituted furyl, the substituted pyridyl and the substituted phenyl are each independently substituted with one or more hydroxyl-C₁₋₆ alkylene groups;
further preferably, R is selected from the group consisting of pyridin-1-yl, pyridin-2-yl, pyridin-3-yl, pyridin-4-yl, p-hydroxymethylphenyl, o-hydroxymethylphenyl, m-hydroxymethylphenyl, p-hydroxyethylphenyl, o-hydroxyethylphenyl, m-hydroxyethylphenyl, p-hydroxypropylphenyl, cyclopropyl and cyclobutyl;
more preferably, R is selected from

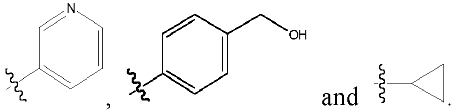

3. The compound, its pharmaceutically acceptable salt, its stereoisomer, its pharmaceutically acceptable hydrate or solvate, or its pharmaceutically acceptable ester according to claim 1 or 2, which is selected from the following compounds, their pharmaceutically acceptable salts or their stereoisomers: 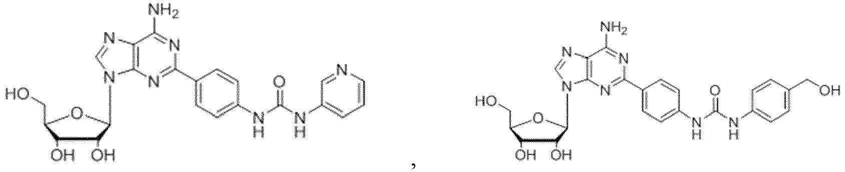 and

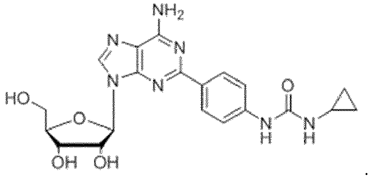

4. A pharmaceutical composition, which comprises the compound, its pharmaceutically acceptable salt, its stereoisomer, its pharmaceutically acceptable hydrate or solvate, or its pharmaceutically acceptable ester according to any one of claims 1 to 3, and optionally a pharmaceutical excipient;
preferably, the pharmaceutical composition is an A_{2A} adenosine receptor agonist.

5. A method for agonizing A_{2A} adenosine receptor, comprising administering an effective amount of the compound, its pharmaceutically acceptable salt, its stereoisomer, its pharmaceutically acceptable hydrate or solvate, or its pharmaceutically acceptable ester according to any one of claims 1 to 3, or an effective amount of the pharmaceutical composition according to claim 4 on an A_{2A} adenosine receptor.

6. A method for improving the permeability of blood-brain barrier, comprising administering an effective amount of the compound, its pharmaceutically acceptable salt, its stereoisomer, its pharmaceutically acceptable hydrate or solvate, or its pharmaceutically acceptable ester according to any one of claims 1 to 3, or an effective amount of the pharmaceutical composition according to claim 4 to a subject.

7. A method for promoting the delivery of a drug across blood-brain barrier, comprising improving the permeability of blood-brain barrier by the method according to claim 6, and then administering the drug to the subject; or, administering the drug together with an effective amount of the compound, its pharmaceutically acceptable salt, its stereoisomer, its pharmaceutically acceptable hydrate or solvate, or its pharmaceutically acceptable ester according to any one of claims 1 to 3, or the drug together with an effective amount of the pharmaceutical composition according to claim 4 to the subject.

8. A method for preventing or treating a disease associated with A_{2A} adenosine receptor agonistic activity, comprising administering an effective amount of the compound, its pharmaceutically acceptable salt, its stereoisomer, its pharmaceutically acceptable hydrate or solvate, or its pharmaceutically acceptable ester according to any one of claims 1 to 3, or an effective amount of the pharmaceutical composition according to claim 4 to a subject in need;
preferably, the disease associated with A_{2A} adenosine receptor agonistic activity is selected from the group consisting of autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

9. Use of the compound, its pharmaceutically acceptable salt, its stereoisomer, its pharmaceutically acceptable hydrate or solvate, or its pharmaceutically acceptable ester according to any one of claims 1 to 3, or the pharmaceutical composition according to claim 4 in the manufacture of an A_{2A} adenosine receptor agonist or a medicament for improving the permeability of blood-brain barrier.

10. Use of the compound, its pharmaceutically acceptable salt, its stereoisomer, its pharmaceutically acceptable hydrate or solvate, or its pharmaceutically acceptable ester according to any one of claims 1 to 3, or the pharmaceutical composition according to claim 4 in the manufacture of a medicament for preventing or treating a disease associated with A_{2A} adenosine receptor agonistic activity;
preferably, the disease associated with A_{2A} adenosine receptor agonistic activity is selected from the group consisting of autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

11. Use of the compound, its pharmaceutically acceptable salt, its stereoisomer, its pharmaceutically acceptable hydrate or solvate, or its pharmaceutically acceptable ester according to any one of claims 1 to 3, or the pharmaceutical composition according to claim 4 in the manufacture of a medicament for diagnosing a myocardial perfusion abnormality disease or in the manufacture of a vasodilator.

12. A method for diagnosing myocardial perfusion abnormality disease, comprising administering a diagnostically effective amount of the compound, its pharmaceutically acceptable salt, its stereoisomer, its pharmaceutically acceptable hydrate or solvate, or its pharmaceutically acceptable ester according to any one of claims 1 to 3, or a diagnostically effective amount of the pharmaceutical composition according to claim 4 to a subject in need.

13. The compound, its pharmaceutically acceptable salt, its stereoisomer, its pharmaceutically acceptable hydrate or solvate, or its pharmaceutically acceptable ester according to any one of claims 1 to 3, or the pharmaceutical composition according to claim 4, for use in agonizing A_{2A} adenosine receptor, improving the permeability of blood-brain barrier or promoting delivery of drug across blood-brain barrier.

14. The compound, its pharmaceutically acceptable salt, its stereoisomer, its pharmaceutically acceptable hydrate or solvate, or its pharmaceutically acceptable ester according to any one of claims 1 to 3, or the pharmaceutical composition according to claim 4, for use in preventing or treating a disease associated with A_{2A} adenosine receptor agonistic activity;
preferably, the disease associated with A_{2A} adenosine receptor agonistic activity is selected from the group consisting of autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, foundrymen's disease, glaucoma, ocular hypertension, spinal cord injury, myocardial infarction, and acute myocardial infarction.

15. The compound, its pharmaceutically acceptable salt, its stereoisomer, its pharmaceutically acceptable hydrate or solvate, or its pharmaceutically acceptable ester according to any one of claims 1 to 3, or the pharmaceutical composition according to claim 4, for use in diagnosing an myocardial perfusion abnormality disease or dilating a blood vessel.

16. A pharmaceutical composition, which comprises the compound, its pharmaceutically acceptable salt, its stereoisomer, its pharmaceutically acceptable hydrate or solvate, or its pharmaceutically acceptable ester according to any one of claims 1 to 3, and a drug suitable for delivery across blood-brain barrier and optionally a pharmaceutical excipient.
